# EUROPÄISCHE PATENTANMELDUNG

(11) **EP 4 524 088 A1**
(43) Veröffentlichungstag der Anmeldung: **19.03.2025**
(21) Anmeldenummer: 24200830.8
(22) Anmeldetag: 17.09.2024
(51) Int. Cl.: B67C 3/00, B65C 9/40, F16P 3/08, F16P 3/12, B65B 59/00

(54) **BEHÄLTERBEHANDLUNGSANLAGE MIT VERBESSERTEM PERSONENSCHUTZ**

(30) Priorität: 18.09.2023 DE 202023105407 U
(71) Anmelder: KRONES AG, 93073 Neutraubling (DE)
(72) Erfinder: EHRISMANN, Tobias, 93073 Neutraubling (DE); SCHERL, Stefan, 93073 Neutraubling (DE)
(74) Vertreter: v. Bezold & Partner Patentanwälte - PartG mbB

(57) **Zusammenfassung**

Die Erfindung betrifft eine Behälterbehandlungsanlage (10) aufweisend eine Behälterbehandlungsvorrichtung (12) mit einem Behandlungsbereich (14) zum Behandeln von Behältern und einer Schutzeinhausung (16), die den Behandlungsbereich (14) zumindest teilweise einhaust. Die Behälterbehandlungsanlage (10) weist ferner eine Sensorvorrichtung (30) auf, die dazu konfiguriert ist, ein Eindringen in einen vorgegebenen Schutzbereich (32) in einer Umgebung der Behälterbehandlungsvorrichtung (12) zu erfassen. Die Behälterbehandlungsanlage (10) weist ferner eine Verarbeitungsvorrichtung (38) auf, die dazu konfiguriert ist, abhängig von einer Signalausgabe der Sensorvorrichtung (30) einen Betrieb der Behälterbehandlungsvorrichtung (12) und/oder der Behälterbehandlungsanlage (10) anzupassen.

## Beschreibung

### Technisches Gebiet

Die Erfindung betrifft eine Behälterbehandlungsanlage mit einer Behälterbehandlungsvorrichtung, die eine Schutzeinhausung aufweist.

### Technischer Hintergrund

In einer Behälterbehandlungsanlage kann eine Behälterbehandlungsvorrichtung mit einem physischen Maschinenschutz (Schutzeinhausung) ausgestattet sein. Dieser Maschinenschutz soll verhindern, dass sich Personen während der Produktion direkt an der Behälterbehandlungsvorrichtung durch sich bewegende Teile verletzen, z. B. durch Quetschungen oder Verbrennungen. Der Maschinenschutz kann ebenfalls verhindern, dass ungewünscht Teile bzw. Medien aus der Behälterbehandlungsvorrichtung austreten, die ebenfalls Personen verletzen könnten.

Die DE 20 2018 101 090 U1 offenbart eine Etikettiermaschine umfassend ein Karussell und ein an der Peripherie des Karussells austauschbar anordenbares Etikettieraggregat. Das Etikettieraggregat umfasst einen feststehenden Unterbau, ein bezüglich des Unterbaus in Richtung Karussell verschiebbares Funktionsteil und einen Aggregatschutz, der im angekoppelten Zustand des Etikettieraggregats den Bereich der Etikettenübergabe vor Benutzereingriffen schützt. Der Aggregatschutz weist mindestens ein am Unterbau ortsfest angebrachtes Schutzelement und ein gegenüber diesem Schutzelement in Richtung Karussell verschiebbares Schutzelement auf.

Die DE 10 2017 215 467 A1 offenbart eine Behälterbehandlungsanlage umfassend einen Maschinenbereich, der zumindest teilweise durch eine Verschutzung eingehaust ist, und ein durch eine Öffnung in der Verschutzung hindurch mit dem Maschinenbereich zusammenarbeitendes Aggregat.

Nachteilig an der herkömmlichen Schutztechnik kann sein, dass der Maschinenschutz außerhalb der Betriebszeiten, z. B. bei einem Umrüst- oder Wechselvorgang, oftmals nicht aktiv bleiben kann, da ein Eingriff von außen notwendig ist bzw. gewisse Materialien den Maschinenschutz "durchqueren" müssen. Daher muss dann bspw. eine Tür oder ein Schutzblech des Maschinenschutzes geöffnet werden. Das birgt vor allem bei einem halb- oder vollautomatischen Wechselbetrieb, bei dem eigentlich keine Person anwesend sein müsste, die Gefahr von Verletzungen, da ein Hineingreifen durch die Person nicht ausgeschlossen werden kann. Selbst bei sehr langsamen Bewegungen der Behälterbehandlungsvorrichtung kann ein Restrisiko von Verletzungen nicht ausgeschlossen werden.

Der Erfindung liegt die Aufgabe zu Grunde, eine verbesserte Technik zum Schützen von Personen vor Verletzungen an einer Behälterbehandlungsvorrichtung zu schaffen.

### Zusammenfassung der Erfindung

Die Aufgabe wird gelöst durch die Merkmale des unabhängigen Anspruchs 1. Vorteilhafte Weiterbildungen sind in den abhängigen Ansprüchen und der Beschreibung angegeben.

Ein Aspekt der vorliegenden Offenbarung betrifft eine Behälterbehandlungsanlage. Die Behälterbehandlungsanlage weist eine Behälterbehandlungsvorrichtung (z. B. Behälteretikettiervorrichtung) mit einem (z. B. innenliegenden) Behandlungsbereich zum Behandeln von Behältern und einer (z. B. äußeren) Schutzeinhausung, die den Behandlungsbereich zumindest teilweise einhaust, auf. Die Behälterbehandlungsanlage weist eine, vorzugsweise optische und/oder selektiv zuschaltbare, Sensorvorrichtung auf, die dazu konfiguriert ist, ein Eindringen (z. B. eines Objekts, wie eine Person) in einen vorgegebenen, vorzugsweise selektiv zuschaltbaren, Schutzbereich in einer Umgebung der Behälterbehandlungsvorrichtung zu erfassen. Die Behälterbehandlungsanlage weist eine Verarbeitungsvorrichtung auf, die dazu konfiguriert ist, abhängig von einer Signalausgabe (z. B. bezüglich des Erfassens des Eindringens in den Schutzbereich) der Sensorvorrichtung einen Betrieb der Behälterbehandlungsvorrichtung und/oder der Behälterbehandlungsanlage anzupassen.

Vorteilhaft kann der Schutzbereich in bestimmten Situationen zugeschaltet bzw. aktiviert werden und dann bspw. ergänzend, zusätzlich oder anstelle der Schutzeinhausung wirken. So kann beispielsweise bei einer automatischen Umrüstung der Behälterbehandlungsvorrichtung mit einem automatischen Formatteilewechsel, bei dem die physische Schutzeinhausung geöffnet ist und die Behälterbehandlungsvorrichtung sich drehen muss, der (weiträumige) Schutzbereich aktiviert werden, um eine Gefährdung von Bedienpersonal zumindest zu verringern. Vorteilhaft kann die Vorrichtung auch ermöglichen, automatisch durchgeführte Aufgaben von z. B. Robotern zu parallelisieren und mehrere Aufgaben von Bediener und Automatik simultan durchführen zu können, ohne die Sicherheit des Bedieners zu gefährden, da dieser bspw. nur außerhalb des Schutzbereichs tätig sein darf. Vorteilhaft können auf diese Weise schnellere Umrüstzeiten und eine höhere Gesamtlinieneffizienz ermöglicht werden.

In einem Ausführungsbeispiel weist die Schutzeinhausung einen öffenbaren Abschnitt, vorzugsweise eine Tür, ein Fenster, eine Klappe oder eine Schleuse, zum Zugänglichmachen des Behandlungsbereichs von außerhalb der Schutzeinhausung auf. Optional kann die Sensorvorrichtung dazu konfiguriert sein, dass der Schutzbereich derart vorgegeben ist, dass er an den öffenbaren Abschnitt angrenzt. Vorteilhaft kann damit insbesondere das von dem geöffneten Abschnitt ausgehende Gefährdungspotenzial für Personen in der Behälterbehandlungsanlage verringert werden.

Vorzugsweise kann die Sensorvorrichtung dazu konfiguriert sein, dass der Schutzbereich derart vorgegeben ist, dass er die Behälterbehandlungsvorrichtung und/oder den öffenbaren Abschnitt zumindest teilweise umhüllt.

In einem weiteren Ausführungsbeispiel ist die Verarbeitungsvorrichtung dazu konfiguriert, die Sensorvorrichtung und/oder den Schutzbereich zuzuschalten (z. B. die Sensorvorrichtung zu aktivieren und/oder eine Verarbeitung der Signalausgabe der Sensorvorrichtung zu aktivieren), wenn oder bevor der öffenbare Abschnitt, vorzugsweise automatisch, geöffnet wird. Vorteilhaft kann damit der Schutzbereich nur dann zugeschaltet werden, wenn tatsächlich ein Gefährdungspotential für Personen in der Anlage besteht.

In einem weiteren Ausführungsbeispiel weist Behälterbehandlungsanlage ferner ein Wechselautomat auf, der zum automatischen Wechseln, Warten oder Umrüsten von (z. B. Rüst- und/oder Format- und/oder Garnitur-) Teilen in dem Behandlungsbereich der Behälterbehandlungsvorrichtung ausgebildet ist. Vorzugsweise kann der Wechselautomat außerhalb von der Schutzeinhausung angeordnet und dazu ausgebildet sein, durch den öffenbaren Abschnitt hindurch die Teile in dem Behandlungsbereich der Behälterbehandlungsvorrichtung zu wechseln, zu warten oder umzurüsten. Vorteilhaft kann der zugeschaltete Schutzbereich somit beispielsweise eine Gefährdung von Personen durch den Wechselautomaten und die Behälterbehandlungsvorrichtung während des Umrüstens usw. verhindern.

In einer Ausführungsform ist die Verarbeitungsvorrichtung dazu konfiguriert, die Sensorvorrichtung und/oder den Schutzbereich zuzuschalten (z. B. die Sensorvorrichtung zu aktivieren oder eine Verarbeitung der Signalausgabe der Sensorvorrichtung zu aktivieren), wenn oder bevor der Wechselautomat zum automatischen Wechseln, Warten oder Umrüsten der Teile in dem Behandlungsbereich der Behälterbehandlungsvorrichtung (z. B. manuell oder automatisch) aktiviert wird. Vorteilhaft kann der Schutzbereich somit besonders zuverlässig eine Gefährdung von Personen durch den Wechselautomaten verringern oder verhindern.

In einer weiteren Ausführungsform weist die Behälterbehandlungsanlage ferner eine Benutzerschnittstelle (z. B. eine freistehende Touchsäule) auf, die außerhalb von dem vorgegebenen Schutzbereich angeordnet ist und über die die Sensorvorrichtung und/oder der Schutzbereich zuschaltbar ist. Vorzugsweise kann die Benutzerschnittstelle eine Anlagenbenutzerschnittstelle oder eine Benutzerschnittstelle einer weiteren Behälterbehandlungsvorrichtung (z. B. Füllvorrichtung, Verschließvorrichtung oder Behälterherstellvorrichtung) der Behälterbehandlungsanlage sein. Vorteilhaft kann damit erreicht werden, dass der Schutzbereich nur von außerhalb des Schutzbereichs zuschaltbar ist. Vorteilhaft besteht somit nicht die Gefahr, dass eine sich bereits im Schutzbereich und damit im Gefährdungsbereich aufhaltende Person den Schutzbereich aktiviert, da hier - je nach Ausführung der Sensorvorrichtung - gar kein Eindringen in den Schutzbereich von außen mehr erfassbar wäre.

In einer Ausführungsvariante weist die Behälterbehandlungsanlage ferner eine weitere Benutzerschnittstelle auf, die innerhalb von dem vorgegebenen Schutzbereich angeordnet ist und über die die Sensorvorrichtung und/oder der Schutzbereich nicht zuschaltbar ist, wobei vorzugsweise die weitere Benutzerschnittstelle eine Benutzerschnittstelle der Behälterbehandlungsvorrichtung ist. Vorteilhaft kann damit das Schutzkonzept zum Verhindern des Zuschaltens des Schutzbereichs von innerhalb des Schutzbereichs weiter verbessert werden.

In einer weiteren Ausführungsvariante weist die Sensorvorrichtung mindestens eine Kamera und/oder mindestens einen Signalscanner, vorzugsweise Laserscanner, auf.

In einem Ausführungsbeispiel ist die Sensorvorrichtung dazu konfiguriert, mindestens einen (z. B. vertikal ausgerichteten oder horizontal ausgerichteten) Signalvorhang, vorzugsweise Licht- oder Laservorhang; und/oder mindestens eine Signalschranke, vorzugsweise Licht- oder Laserschranke; und/oder mindestens ein (z. B. vertikal oder horizontal ausgerichtetes) Signalgitter, vorzugsweise Lichtgitter oder Lasergitter, zum Erfassen des Eindringens zu erzeugen. Vorteilhaft kann der Signalvorhang, die Signalschranke und das Signalgitter als äußere Begrenzungen des Schutzbereichs dienen, sodass das Eindringen in den Schutzbereich besonders sicher und schnell erkannt werden kann.

In einem weiteren Ausführungsbeispiel ist die Sensorvorrichtung dazu konfiguriert, mehrere Signalvorhänge, mehrere Signalschranken oder mehrere Signalgitter derart zu erzeugen, dass die mehreren Signalvorhänge, die mehreren Signalschranken oder die mehreren Signalgitter winkelig zueinander angeordnet sind und vorzugsweise aneinander angrenzen oder sich schneiden. Vorteilhaft kann damit eine zumindest abschnittsweise Umhüllung der Behälterbehandlungsvorrichtung durch den Schutzbereich ermöglicht werden.

In einer Ausführungsform weist die Sensorvorrichtung einen Emitter, vorzugsweise Laseremitter, auf, wobei der Emitter dazu konfiguriert ist, einen, vorzugsweise kreisförmigen oder fächerförmigen, (z. B. vertikal oder horizontal ausgerichteten) Signalvorhang, vorzugsweise Laservorhang, auszugeben. Die Sensorvorrichtung weist ferner einen Empfänger, vorzugsweise Laserempfänger, auf, wobei der Empfänger dazu konfiguriert ist, zu erfassen, wenn ein Signalstrahl, vorzugsweise Laserstrahl, des Signalvorhangs an einem Objekt rückreflektiert wird. Vorteilhaft kann damit eine Technik genutzt werden, bei der der Signalvorhang wie gewünscht frei im Raum positioniert werden kann, da keine Beschränkungen aufgrund der Anordnung des Empfängers bestehen.

Vorzugsweise kann der Empfänger direkt neben dem Emitter angeordnet sein.

Es ist auch möglich, dass die Sensorvorrichtung einen weiteren Emitter, vorzugsweise Laseremitter, aufweist, wobei der weitere Emitter dazu konfiguriert ist, einen weiteren, vorzugsweise kreisförmigen oder fächerförmigen, (z. B. vertikal oder horizontal ausgerichteten) Signalvorhang, vorzugsweise Laservorhang, auszugeben. Die Sensorvorrichtung kann ferner einen weiteren Empfänger, vorzugsweise Laserempfänger, aufweisen, wobei der weitere Empfänger dazu konfiguriert ist, zu erfassen, wenn ein Signalstrahl, vorzugsweise Laserstrahl, des weiteren Signalvorhangs an einem Objekt rückreflektiert wird.

Vorzugsweise können der Signalvorhang und der weitere Signalvorhang winkelig zueinander ausgerichtet sein und bspw. aneinander angrenzen oder sich schneiden.

In einer weiteren Ausführungsform ist die Verarbeitungsvorrichtung dazu konfiguriert, den Betrieb abhängig von der Signalausgabe derart anzupassen, dass ein automatischer Umrüst-, Teilewechsel- oder Wartungsbetrieb der Behälterbehandlungsvorrichtung gestoppt wird und/oder ein Warnsignal ausgegeben wird, wenn die Signalausgabe der Sensorvorrichtung das Eindringen in den vorgegebenen Schutzbereich angibt, wobei vorzugsweise in dem automatischen Umrüst-, Teilewechsel- oder Wartungsbetrieb ein öffenbarer Abschnitt der Schutzeinhausung geöffnet ist. Vorteilhaft kann der zugeschaltete Schutzbereich somit eine Gefährdung von Personen während des Umrüst-, Teilewechsel- oder Wartungsbetriebs verhindern.

In einer Ausführungsvariante ist die Verarbeitungsvorrichtung dazu konfiguriert, dass die Sensorvorrichtung, der Schutzbereich und/oder eine Verarbeitung der Signalausgabe der Sensorvorrichtung deaktiviert ist, wenn die Behälterbehandlungsvorrichtung in einem (z. B. Normal-) Betriebsmodus betrieben wird, in dem die Schutzeinhausung und/oder ein öffenbarer Abschnitt der Schutzeinhausung (z. B. vollständig) geschlossen (ungeöffnet) ist. Vorteilhaft kann damit ein Aufenthalt einer Person nahe der Behälterbehandlungsvorrichtung während des Normalbetriebs zum Überwachen der Behälterbehandlung zugelassen sein, da hier die Schutzeinhausung die Personen sicher schützen kann.

In einer weiteren Ausführungsvariante ist die Sensorvorrichtung dazu konfiguriert, ein Eindringen und/oder Aufenthalt (z. B. eines Objekts, wie einer Person) in einem vorgegebenen sicheren Zugangskorridor in dem vorgegebenen Schutzbereich zu erfassen, oder die Sensorvorrichtung ist dazu konfiguriert, ein Eindringen und/oder Aufenthalt in einem vorgegebenen sicheren Zugangskorridor in dem vorgegebenen Schutzbereich nicht als ein Eindringen in den vorgegebenen Schutzbereich zu erfassen. Vorteilhaft kann somit ein Korridor in dem Schutzbereich ausgespart werden, sodass dem Bediener ein gewisser Zugang zu notwendigen Punkten nahe der Behälterbehandlungsvorrichtung gestattet werden kann, um beispielsweise die automatische Umrüstung zu überwachen. Dafür bieten sich bspw. Kamera- oder Scanner-Schutzkonzepte besonders an.

In einem weiteren Ausführungsbeispiel weist die Behälterbehandlungsanlage ferner mindestens eine weitere Behälterbehandlungsvorrichtung auf, die neben der Behälterbehandlungsvorrichtung angeordnet und bspw. mit der Behälterbehandlungsvorrichtung zum Transportieren von Behältern verbundenen ist. Die Sensorvorrichtung kann vorzugsweise dazu konfiguriert sein, dass der Schutzbereich derart vorgegeben ist, dass die mindestens eine weitere Behälterbehandlungsvorrichtung den Schutzbereich begrenzt. Vorteilhaft können somit auch andere Maschinen in der Anlage als physische Außengrenzen des Schutzbereichs genutzt werden, sodass bspw. Sensortechnik eingespart werden kann.

Vorzugsweise kann die Behälterbehandlungsanlage zum Temperieren, Herstellen, Reinigen, Beschichten, Prüfen, Abfüllen, Verschließen, Pasteurisieren, Etikettieren, Bedrucken, Beschriften, Laserbeschriften und/oder Verpacken von Behältern für flüssige oder pastöse Medien, vorzugsweise Getränke, flüssige Nahrungsmittel oder Produkte der Pharma- oder HealthCare Industrie , ausgebildet sein.

Beispielsweise können die Behälter als Flaschen, Dosen, Kanister, Kartons, Flakons, Tuben usw. ausgeführt sein.

Vorzugsweise kann sich der Begriff "Verarbeitungsvorrichtung" auf eine Elektronik (z. B. ausgeführt als eine Treiberschaltung oder mit Mikroprozessor(en) und Datenspeicher) beziehen, die je nach Ausbildung Steuerungsaufgaben und/oder Regelungsaufgaben und/oder Verarbeitungsaufgaben übernehmen kann. Auch wenn hierin der Begriff "Steuern" verwendet wird, kann damit gleichsam zweckmäßig auch "Regeln" bzw. "Steuern mit Rückkopplung" und/oder "Verarbeiten" umfasst bzw. gemeint sein.

Die zuvor beschriebenen bevorzugten Ausführungsformen und Merkmale der Erfindung sind beliebig miteinander kombinierbar.

### Kurzbeschreibung der Figuren

Weitere Einzelheiten und Vorteile der Erfindung werden im Folgenden unter Bezug auf die beigefügten Zeichnungen beschrieben. Es zeigen:
- Figur 1: eine perspektivische Ansicht einer schematisch dargestellten Behälterbehandlungsanlage gemäß einem Ausführungsbeispiel der vorliegenden Offenbarung; und
- Figur 2: eine Draufsicht auf die Behälterbehandlungsanlage von Figur 1.

Die in den Figuren gezeigten Ausführungsformen stimmen zumindest teilweise überein, so dass ähnliche oder identische Teile mit den gleichen Bezugszeichen versehen sind und zu deren Erläuterung auch auf die Beschreibung der anderen Ausführungsformen bzw. Figuren verwiesen wird, um Wiederholungen zu vermeiden.

### Detaillierte Beschreibung beispielhafter Ausführungsformen

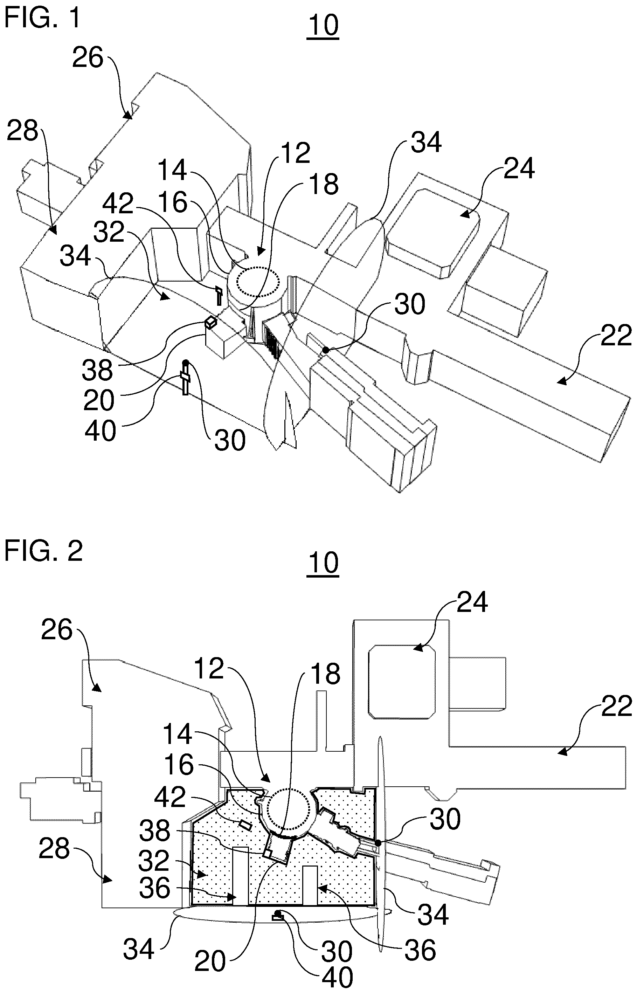

Die Figuren 1 und 2 zeigen eine Behälterbehandlungsanlage 10.

Die Behälterbehandlungsanlage 10 weist eine Behälterbehandlungsvorrichtung 12, eine Sensorvorrichtung 30 und eine Verarbeitungsvorrichtung 38 auf. Optional kann die Behälterbehandlungsanlage 10 bspw. noch ein Wechselautomat 20, mindestens eine weitere Behälterbehandlungsvorrichtung 22, 24, 26, 28 und/oder mindestens eine Benutzerschnittstelle 40, 42 aufweisen.

Die Behälterbehandlungsvorrichtung 12 ist zum Behandeln von Behältern ausgeführt. Im dargestellten bevorzugten Ausführungsbeispiel ist die Behälterbehandlungsvorrichtung 12 als eine Etikettiervorrichtung ausgebildet.

Die Etikettiervorrichtung kann Behälter etikettieren. Beispielsweise kann die Etikettiervorrichtung mindestens ein Etikettieraggregat aufweisen. Das mindestens eine Etikettieraggregat kann austauschbar an einer Peripherie der Etikettiervorrichtung an- und abkoppelbar sein, z. B. über einen, vorzugsweise werkzeuglosen, Schnellkoppelmechanismus. Das mindestens eine Etikettieraggregat kann zur Applikation von Etiketten, z. B. Selbstklebeetiketten, Kaltleimetiketten oder Rollenetiketten, ausgebildet sein.

Es ist möglich, dass die Behälterbehandlungsvorrichtung 12 für eine andere Art von Behälterbehandlung ausgeführt ist. Bspw. kann die Behälterbehandlungsvorrichtung 12 dazu ausgebildet sein, die Behälter zu temperieren, herzustellen, zu reinigen, zu beschichten, zu prüfen, zu füllen, zu verschließen, zu pasteurisieren, zu bedrucken, zu beschriften, zu laserbeschriften oder zu verpacken. Die Behälterbehandlungsvorrichtung 12 kann beispielsweise ein Preform-Heizofen (Vorformling-Heizofen), eine Behälterherstellvorrichtung (z. B. Behälterblasmaschine), eine Beschichtungsvorrichtung, eine Entpalletiervorrichtung, eine Rinsvorrichtung (z. B. mit Rinsdüsen), eine Sterilisierungsvorrichtung (z. B. mit Sterilisationsdüsen), eine Füllvorrichtung (z. B. mit Füllventilen), eine Verschließvorrichtung (z. B. mit Verschließköpfen), eine Beschriftungsvorrichtung (z. B. mit Lasermarkern), eine Druckvorrichtung (z. B. mit Druckköpfen), eine Pasteurisiervorrichtung, eine Klebegebindeherstellvorrichtung (z. B. mit Klebstoffapplikationsdüsen), eine Verpackungsvorrichtung (z. B. mit Packköpfen oder Folieneinschlägern) und/oder eine Palettiervorrichtung (z. B. mit Palettierköpfen, Schiebeplatten oder dgl.) sein.

Die Behälterbehandlungsvorrichtung 12 kann mittels eines Antriebs drehbar sein. Vorzugsweise kann die Behälterbehandlungsvorrichtung 12 als eine Rundläufer- Behälterbehandlungsvorrichtung mit einem mittels des Antriebs drehbaren Karussell ausgeführt sein. Behälter können beabstandet voneinander an einem Umfang des Karussells in Behandlungsstationen gehalten und behandelt, z. B. etikettiert, werden. Alternativ kann die Behälterbehandlungsvorrichtung 12 bspw. als eine Linear-Behälterbehandlungsvorrichtung ausgeführt sein, z. B. mit nur einer Behandlungsstation oder mit mehreren in Reihe nebeneinander und/oder hintereinander angeordneten Behandlungsstationen.

Die Behälterbehandlungsvorrichtung 12 kann mit der mindestens einen weiteren Behälterhandlungsvorrichtung 22, 24, 26, 28 mittels eines Behälterförderersystems verbunden sein. Das Behälterförderersystem kann bspw. mindestens einen Transportstern und/oder mindestens einen Linearförderer aufweisen.

Die Behälterbehandlungsvorrichtung 12 weist einen Behandlungsbereich 14 und eine mechanische Schutzeinhausung 16 auf.

Im Behandlungsbereich 14 werden die Behälter abhängig von der Ausführung der Behälterbehandlungsvorrichtung 12 behandelt, z. B. etikettiert usw.

Die Schutzeinhausung 16 haust den Behandlungsbereich 14 teilweise, im Wesentlichen vollständig oder vollständig ein. Der Behandlungsbereich 14 kann geschützt innerhalb der Schutzeinhausung 16 angeordnet sein.

Die Schutzeinhausung 16 kann bspw. Wände, Fenster usw. aufweisen, um den Behandlungsbereich 14 einzuhausen. Die Schutzeinhausung 16 kann bspw. im Wesentlichen eine zylindermantelförmige oder im Wesentlichen eine zylindermantelsegmentförmige Außenwand und optional ein Dach aufweisen.

Bevorzugt kann die Schutzeinhausung 16 einen öffenbaren Abschnitt 18 aufweisen. Wenn der Abschnitt 18 geöffnet ist, kann er einen Zugang von außerhalb der Schutzeinhausung 16 zu dem Behandlungsbereich 14 bereitstellen. Der Abschnitt 18 kann beispielsweise eine Tür, ein Fenster, eine Klappe oder eine Schleuse sein.

Die Schutzeinhausung 16 kann mit einer optionalen Schutzeinhausung der mindestens einen weiteren Behälterbehandlungsvorrichtung 22, 24, 26, 28 verbunden sein. Beispielsweise kann die Schutzeinhausung 16 in eine Schutzeinhausung der Behälterbehandlungsvorrichtung 24 und/oder 26 übergehen.

Der Wechselautomat 20 ist zum automatischen Wechseln, Warten oder Umrüsten von Teilen in dem Behandlungsbereich 14 der Behälterbehandlungsvorrichtung 12 ausgebildet. Die Teile sind vorzugsweise Rüstteile, wie z. B. Behälterhalterungen, Behälterdrehteller, Greifer, Zentrierglocken usw., oder jegliche anderen Teile, z. B. Ersatzteile. Beim Umrüsten können bspw. Rüstteile der Behälterbehandlungsvorrichtung 12 ausgetauscht werden, um die Behälterbehandlungsvorrichtung 12 an ein anderes Behälterformat anzupassen.

Vorzugsweise weist der Wechselautomat 20 eine Handhabungseinrichtung zum Ausführen von Arbeiten in dem Behandlungsbereich 14 auf. Vorzugsweise kann die Handhabungseinrichtung mindestens ein Werkzeug, wie bspw. Greifer, zum Ausführen der Arbeiten aufweisen.

Die Handhabungseinrichtung kann bspw. ein stationärer oder mobiler Roboter (z. B. Wechselroboter) oder jegliche andere Art von Handhabungseinrichtung sein. Vorzugsweise kann die Handhabungseinrichtung mindestens einen verschwenkbaren und/oder verschiebbaren Arm aufweisen.

Es ist möglich, dass der Wechselautomat 20 ein Magazin aufweist. Im Magazin können Teile, wie z. B. Ersatzteile oder Rüstteile, aufgenommen sein. Vorzugsweise kann die Handhabungseinrichtung des Wechselautomaten 20 die Teile von dem Magazin zu dem Behandlungsbereich 14 bewegen.

Bevorzugt ist der Wechselautomat 20 außerhalb von der Schutzeinhausung 16 angeordnet. Der Wechselautomat 20 kann durch den geöffneten Abschnitt 18 hindurch die Teile in dem Behandlungsbereich 14 wechseln, warten oder umrüsten. Beispielsweise kann die Handhabungseinrichtung, z. B. deren mindestens einer Arm und/oder deren mindestens eines Werkzeugs, durch den geöffneten Abschnitt 18 hindurch die Teile in dem Behandlungsbereich 14 wechseln, warten oder umrüsten.

Der Wechselautomat 20 kann vorzugsweise in einem automatischen Umrüst-, Teilewechsel- oder Wartungsbetrieb (bzw. -betriebsmodus) der Behälterbehandlungsvorrichtung 12 betrieben werden. In diesem automatischen Umrüst-, Teilewechsel- oder Wartungsbetrieb kann der Abschnitt 18 geöffnet sein. Im Umrüst-, Teilewechsel- oder Wartungsbetrieb werden vorzugsweise keine Behälter in dem Behandlungsbereich 14 behandelt. D.h., die Behälterbehandlungsvorrichtung 12 ist nicht in einem Normalbetrieb/Produktionsbetrieb.

Wie bereits erwähnt, kann die Behälterbehandlungsanlage 18 mindestens eine weitere Behälterhandlungsvorrichtung 22, 24, 26, 28 aufweisen. Im dargestellten Ausführungsbeispiel ist die weitere Behälterhandlungsvorrichtung 22 bspw. ein Preform-Heizofen. Die Behälterhandlungsvorrichtung 24 kann eine Behälterherstellvorrichtung (z. B. Behälterblasvorrichtung) sein. Die Behälterhandlungsvorrichtung 26 kann eine Füllvorrichtung zum Füllen von Behältern sein. Die Behälterhandlungsvorrichtung 28 kann eine Verschließvorrichtung zum Verschließen von Behältern sein.

Die mindestens eine weitere Behälterhandlungsvorrichtung 22, 24, 26, 28 kann bspw. eine Rundläufer- oder eine Linear-Behälterhandlungsvorrichtung sein.

Die mindestens eine weitere Behälterhandlungsvorrichtung 22, 24, 26, 28 kann neben der Behälterbehandlungsvorrichtung 12 angeordnet sein.

Die Sensorvorrichtung 30 ist dazu konfiguriert, ein Eindringen in einen vorgegebenen Schutzbereich 32 in einer Umgebung der Behälterbehandlungsvorrichtung 12 zu erfassen. Beispielsweise kann eine Person in den vorgegebenen Schutzbereich 32 laufen und damit in diesen eindringen. Eine Signalausgabe der Sensorvorrichtung 30 an die Verarbeitungsvorrichtung 38 kann angeben, dass ein Eindringen in den vorgegebenen Schutzbereich 32 von der Sensorvorrichtung 30 erfasst wurde.

Der Schutzbereich 32 kann durch eine Anordnung und Ausrichtung der Sensorvorrichtung 30 vorgegeben sein. Der Schutzbereich 32 kann bspw. derart vorgegeben sein, dass er an den öffenbaren Abschnitt 18 und/oder die Schutzeinhausung 16 angrenzt. Der Schutzbereich 32 kann ferner bspw. derart vorgegeben sein, dass er durch die mindestens eine weitere Behälterbehandlungsvorrichtung 24, 26, 28 begrenzt ist.

Die Sensorvorrichtung 30 ist vorzugsweise eine optische Sensorvorrichtung. Bevorzugt kann die Sensorvorrichtung 30 beispielsweise mindestens eine Kamera und/oder mindestens eine Scannervorrichtung, vorzugsweise Laserscanner, aufweisen.

Die mindestens eine Kamera kann bspw. den Schutzbereich 32 aufnehmen. Beispielweise können Bilderkennungsalgorithmen auf die Aufnahme der Kamera angewendet werden. Mittels der Bilderkennungsalgorithmen kann dann ein Eindringen eines Objekts, wie bspw. einer Person, in den Schutzbereich 32 erfasst werden.

Die mindestens eine Laserscannervorrichtung kann beispielsweise mindestens einen Laservorhang erzeugen. Der mindestens einen Laservorhang ist vorzugsweise vertikal ausgerichtet. Der mindestens einen Laservorhang kann den Schutzbereich 32 außenseitig begrenzen.

Beispielsweise kann die Laserscannervorrichtung einen Laseremitter und einen Laserempfänger aufweisen. Der Laseremitter kann dazu konfiguriert sein, einen, vorzugsweise kreisförmigen oder fächerförmigen, Laservorhang auszugeben. Der Laserempfänger kann dazu konfiguriert sein, zu erfassen, wenn ein Signalstrahl des Laservorhangs an einem Objekt rückreflektiert wird. Der Laserempfänger kann vorzugsweise direkt neben dem Laseremitter angeordnet sein.

Es ist möglich, dass das erläuterte Scannerprinzip mittels einer anderen Technik als der Lasertechnik angewendet wird, um allgemein einen, vorzugsweise kreisförmigen oder fächerförmigen, Signalvorhang mittels eines Emitters auszugeben und mittels eines Empfängers zu erfassen, wenn ein Signalstrahl des Signalvorhangs an einem Objekt rückreflektiert wird. Auch hier kann vorzugsweise der Empfänger direkt neben dem Emitter angeordnet sein.

Ein Scannbereich der Scannervorrichtung, vorzugsweise Laserscannervorrichtung, kann bspw. einen Radius aufweisen, der in einem Bereich zwischen 1.000 mm und 15.000 mm, vorzugsweise zwischen 2.500 mm und 10.000 mm, liegt.

Allgemein kann die Sensorvorrichtung 30 bevorzugt dazu konfiguriert sein, mindestens einen Signalvorhang 38 zum Erfassen des Eindringens in den Schutzbereich 32 zu erzeugen. Der Signalvorhang 38 kann beispielsweise ein Lichtvorhang oder ein Laservorhang sein. Alternativ oder zusätzlich kann die Sensorvorrichtung 30 beispielsweise dazu konfiguriert sein, mindestens eine Signalschranke, vorzugsweise Licht- oder Laserschranke, und/oder mindestens ein Signalgitter, vorzugsweise Lichtgitter oder Lasergitter, zum Erfassen des Eindringens in den Schutzbereich 32 zu erzeugen.

Der mindestens eine Signalvorhang 38, die mindestens eine Signalschranke und/oder das mindestens eine Signalgitter können den Schutzbereich 32 außenseitig begrenzen. Ein Eindringen in den Schutzbereich 32 kann bspw. dann erfasst werden, wenn der mindestens eine Signalvorhang 38, die mindestens eine Signalschranke und/oder das mindestens eine Signalgitter von einem Objekt gestört oder unterbrochen wird.

Bevorzugt werden mittels mehrerer Geräte mehrere Signalvorhänge, mehrere Signalschranken und/oder mehrere Signalgitter erzeugt. Diese sind vorzugsweise winkelig zueinander angeordnet sind und grenzen aneinander an oder schneiden sich, wie in den Figuren beispielhaft dargestellt ist.

Beispielsweise kann die Sensorvorrichtung 30 auf einer Höhe in einem Bereich zwischen 2.000 mm und 10.000 mm angeordnet sein.

Wie beispielhaft in Figur 2 dargestellt ist, kann in dem Schutzbereich 32 mindestens ein sicherer Zugangskorridor 36 angeordnet sein. Der mindestens eine sichere Zugangskorridor 36 kann derart vorgegeben sein, dass ein Aufenthalt einer Person in diesem sicheren Zugangskorridor 36 zugelassen ist, um bspw. eine Arbeit des Wechselautomaten 20 zu überwachen, ohne dass eine Verletzungsgefahr für die Person besteht.

Die Sensorvorrichtung 30 kann entsprechend dazu konfiguriert sein, ein Eindringen und/oder Aufenthalt in dem mindestens einen vorgegebenen sicheren Zugangskorridor 36 in dem vorgegebenen Schutzbereich 32 zu erfassen. Beispielsweise kann eine Signalausgabe der Sensorvorrichtung 30 an die Verarbeitungsvorrichtung 38 angeben, dass ein Eindringen und/oder Aufenthalt in dem mindestens einen vorgegebenen sicheren Zugangskorridor 36 von der Sensorvorrichtung 30 erfasst wurde.

Die Verarbeitungsvorrichtung 38 ist dazu konfiguriert, abhängig von einer Signalausgabe bezüglich des Erfassens des Eindringens in den Schutzbereich von der Sensorvorrichtung 30 einen Betrieb der Behälterbehandlungsvorrichtung 12 und/oder der Behälterbehandlungsanlage 10 anzupassen.

Beispielsweise kann die Verarbeitungsvorrichtung 38 den Betrieb der Behälterbehandlungsanlage 10 abhängig von der Signalausgabe derart anpassen, dass ein automatischer Umrüst-, Teilewechsel- oder Wartungsbetrieb mittels des Wechselautomaten 20 gestoppt wird, wenn die Signalausgabe der Sensorvorrichtung 30 das Eindringen in den vorgegebenen Schutzbereich 32 angibt. Beim Stoppen kann beispielsweise der Wechselautomat 20 und/oder die Behälterbehandlungsvorrichtung 12 angehalten werden. Beispielsweise kann ein sich im Vergleich zum Normalbetrieb während des Umrüst-, Teilewechsel- oder Wartungsbetriebs langsam drehendes oder taktweise drehendes Karussell der Behälterbehandlungsvorrichtung 12 gestoppt werden.

Alternativ oder zusätzlich kann beispielsweise ein optisches oder akustisches Warnsignal ausgegeben werden.

Gibt die Signalausgabe von der Sensorvorrichtung 30 hingegen nur ein Erfassen eines Eindringens und/oder eines Aufenthalts in dem sicheren Zugangskorridor 36 und nicht in dem übrigen Schutzbereich 32 an, kann die Verarbeitungsvorrichtung 38 bspw. davon absehen, den Betrieb, z. B. durch Stoppen des automatischen Umrüst-, Wechsel- oder Wartungsbetriebs, anzupassen.

Alternativ kann die Sensorvorrichtung 30 bspw. dazu konfiguriert sein, ein Eindringen und/oder Aufenthalt in einem vorgegebenen sicheren Zugangskorridor 36 in dem vorgegebenen Schutzbereich 32 gar nicht erst als ein Eindringen in den vorgegebenen Schutzbereich 32 zu erfassen. Entsprechend kann dann gar nicht erst eine Signalausgabe, in der ein Erfassen eines Eindringens in den Schutzbereich 32 angegeben ist, von der Sensorvorrichtung 30 erfolgen. Entsprechend erfolgt auch kein Anpassen des Betriebs mittels der Verarbeitungsvorrichtung 38.

Bevorzugt wird die Sensorvorrichtung 30 nur situativ zugeschaltet.

Beispielsweise kann die Verarbeitungsvorrichtung 38 dazu konfiguriert sein, dass die Sensorvorrichtung 30, der Schutzbereich 32 und/oder eine Verarbeitung der Signalausgabe der Sensorvorrichtung 30 deaktiviert ist, wenn die Behälterbehandlungsvorrichtung 12 in einem Betriebsmodus betrieben wird, in dem der öffenbare Abschnitt 18 der Schutzeinhausung 16 geschlossen (ungeöffnet) ist. Dieser Betriebsmodus kann bspw. der Normalbetriebsmodus der Behälterbehandlungsvorrichtung 12 sein.

Vorzugsweise kann die Verarbeitungsvorrichtung 38 dazu konfiguriert sein, die Sensorvorrichtung 30 und/oder den Schutzbereich 32 zuzuschalten, wenn oder bevor der Wechselautomat 20 zum automatischen Wechseln, Warten oder Umrüsten der Teile in dem Behandlungsbereich 14 der Behälterbehandlungsvorrichtung 12 aktiviert wird. Beispielsweise kann eine manuelle oder automatische Aktivierung des Umrüst-, Teilewechsel- oder Wartungsbetriebs auslösen, dass die Verarbeitungsvorrichtung 38 die Sensorvorrichtung 30 zuschaltet.

Das Zuschalten der Sensorvorrichtung 30 kann bspw. ein Aktivieren der Sensorvorrichtung 30 aufweisen. Alternativ oder zusätzlich kann beim Zuschalten ein Aktivieren einer Verarbeitung der Signalausgabe der Sensorvorrichtung 30 mittels der Verarbeitungsvorrichtung 38 erfolgen.

Die Benutzerschnittstelle 40 ist vorzugsweise außerhalb von dem vorgegebenen Schutzbereich 32 angeordnet. Bevorzugt kann über eine Eingabe eines Benutzers an der Benutzerschnittstelle 40 die Sensorvorrichtung 30 und/oder der Schutzbereich 32 zugeschaltet werden, z. B. direkt und/oder durch Aktivieren eines Umrüst-, Teilewechsel- oder Wartungsbetriebs der Behälterbehandlungsvorrichtung 12. Bei der Benutzerschnittstelle 40 kann es sich vorzugsweise um eine Anlagenbenutzerschnittstelle oder eine Benutzerschnittstelle einer der weiteren Behälterbehandlungsvorrichtungen 22, 24, 26 oder 28 handeln.

Hingegen kann die weitere Benutzerschnittstelle 42 innerhalb von dem vorgegebenen Schutzbereich 32 angeordnet sein. Über diese weitere Benutzerschnittstelle 42 ist die Sensorvorrichtung 32 und/oder den Schutzbereich 32 vorzugsweise nicht zuschaltbar, weder direkt noch indirekt. Beispielsweise kann es sich bei der weiteren Benutzerschnittstelle 42 um eine Benutzerschnittstelle der Behälterbehandlungsvorrichtung 12 handeln, mit der sonst ein Normalbetrieb der Behälterbehandlungsvorrichtung 12 überwacht und angepasst werden kann.

Die mindestens eine Benutzerschnittstelle 40, 42 kann bspw. eine, vorzugsweise berührungsempfindliche, Anzeige, einen Lautsprecher, eine Signalleuchte, ein Mikrofon, Knöpfe und/oder Schalter usw. aufweisen.

Die Erfindung ist nicht auf die vorstehend beschriebenen bevorzugten Ausführungsbeispiele beschränkt. Vielmehr ist eine Vielzahl von Varianten und Abwandlungen möglich, die ebenfalls von dem Erfindungsgedanken Gebrauch machen und deshalb in den Schutzbereich fallen. Insbesondere beansprucht die Erfindung auch Schutz für den Gegenstand und die Merkmale der Unteransprüche unabhängig von den in Bezug genommenen Ansprüchen. Insbesondere sind die einzelnen Merkmale des unabhängigen Anspruchs 1 jeweils unabhängig voneinander offenbart. Zusätzlich sind auch die Merkmale der Unteransprüche unabhängig von sämtlichen Merkmalen des unabhängigen Anspruchs 1 und beispielsweise unabhängig von den Merkmalen bezüglich des Vorhandenseins und/oder der Konfiguration der Behälterbehandlungsvorrichtung, des Behandlungsbereichs, der Schutzeinhausung, der Sensorvorrichtung und/oder der Verarbeitungsvorrichtung des unabhängigen Anspruchs 1 offenbart. Alle Bereichsangaben hierin sind derart offenbart zu verstehen, dass gleichsam alle in den jeweiligen Bereich fallenden Werte einzeln offenbart sind, z. B. auch als jeweils bevorzugte engere Außengrenzen des jeweiligen Bereichs.

### Bezugszeichenliste

- 10: Behälterbehandlungsanlage
- 12: Behälterbehandlungsvorrichtung
- 14: Behandlungsbereich
- 16: Schutzeinhausung
- 18: öffenbarer Abschnitt
- 20: Wechselautomat
- 22: weitere Behälterbehandlungsvorrichtung
- 24: weitere Behälterbehandlungsvorrichtung
- 26: weitere Behälterbehandlungsvorrichtung
- 28: weitere Behälterbehandlungsvorrichtung
- 30: Sensorvorrichtung
- 32: Schutzbereich
- 34: Signalvorhang
- 36: Zugangskorridor
- 38: Verarbeitungsvorrichtung
- 40: Benutzerschnittstelle
- 42: weitere Benutzerschnittstelle

## Patentansprüche

1. Behälterbehandlungsanlage (10) aufweisend:
eine Behälterbehandlungsvorrichtung (12) mit einem Behandlungsbereich (14) zum Behandeln von Behältern und einer Schutzeinhausung (16), die den Behandlungsbereich (14) zumindest teilweise einhaust;
eine, vorzugsweise optische und/oder selektiv zuschaltbare, Sensorvorrichtung (30), die dazu konfiguriert ist, ein Eindringen in einen vorgegebenen, vorzugsweise selektiv zuschaltbaren, Schutzbereich (32) in einer Umgebung der Behälterbehandlungsvorrichtung (12) zu erfassen; und
eine Verarbeitungsvorrichtung (38), die dazu konfiguriert ist, abhängig von einer Signalausgabe der Sensorvorrichtung (30) einen Betrieb der Behälterbehandlungsvorrichtung (12) und/oder der Behälterbehandlungsanlage (10) anzupassen.

2. Behälterbehandlungsanlage (10) nach Anspruch 1, wobei:
die Schutzeinhausung (16) einen öffenbaren Abschnitt (18), vorzugsweise eine Tür, ein Fenster, eine Klappe oder eine Schleuse, zum Zugänglichmachen des Behandlungsbereichs (14) von außerhalb der Schutzeinhausung (16) aufweist; und optional
die Sensorvorrichtung (30) dazu konfiguriert, dass der Schutzbereich (32) derart vorgegeben ist, dass er an den öffenbaren Abschnitt (18) angrenzt.

3. Behälterbehandlungsanlage (10) nach Anspruch 2, wobei:
die Verarbeitungsvorrichtung (38) dazu konfiguriert ist, die Sensorvorrichtung (30) und/oder den Schutzbereich (32) zuzuschalten, wenn oder bevor der öffenbare Abschnitt (18), vorzugsweise automatisch, geöffnet wird.

4. Behälterbehandlungsanlage (10) nach einem der vorherigen Ansprüche, ferner aufweisend:
ein Wechselautomat (20), der zum automatischen Wechseln, Warten oder Umrüsten von Teilen in dem Behandlungsbereich (14) der Behälterbehandlungsvorrichtung (12) ausgebildet ist,
wobei der Wechselautomat (20) außerhalb von der Schutzeinhausung (16) angeordnet und dazu ausgebildet ist, durch den öffenbaren Abschnitt (18) hindurch die Teile in dem Behandlungsbereich (14) der Behälterbehandlungsvorrichtung (12) zu wechseln, zu warten oder umzurüsten.

5. Behälterbehandlungsanlage (10) nach Anspruch 4, wobei:
die Verarbeitungsvorrichtung (38) dazu konfiguriert ist, die Sensorvorrichtung (30) und/oder den Schutzbereich (32) zuzuschalten, wenn oder bevor der Wechselautomat (20) zum automatischen Wechseln, Warten oder Umrüsten der Teile in dem Behandlungsbereich (14) der Behälterbehandlungsvorrichtung (12) aktiviert wird.

6. Behälterbehandlungsanlage (10) nach einem der vorherigen Ansprüche, ferner aufweisend:
eine Benutzerschnittstelle (40), die außerhalb von dem vorgegebenen Schutzbereich (32) angeordnet ist und über die die Sensorvorrichtung (30) und/oder der Schutzbereich (32) zuschaltbar ist,
wobei vorzugsweise die Benutzerschnittstelle (40) eine Anlagenbenutzerschnittstelle oder eine Benutzerschnittstelle einer weiteren Behälterbehandlungsvorrichtung (22, 24, 26, 28) der Behälterbehandlungsanlage (10) ist.

7. Behälterbehandlungsanlage (10) nach Anspruch 6, ferner aufweisend:
eine weitere Benutzerschnittstelle (42), die innerhalb von dem vorgegebenen Schutzbereich (32) angeordnet ist und über die die Sensorvorrichtung (30) und/oder der Schutzbereich (32) nicht zuschaltbar ist,
wobei vorzugsweise die weitere Benutzerschnittstelle (42) eine Benutzerschnittstelle der Behälterbehandlungsvorrichtung (12) ist.

8. Behälterbehandlungsanlage (10) nach einem der vorherigen Ansprüche, wobei:
die Sensorvorrichtung (30) mindestens eine Kamera und/oder mindestens einen Signalscanner, vorzugsweise Laserscanner, aufweist.

9. Behälterbehandlungsanlage (10) nach einem der vorherigen Ansprüche, wobei:
die Sensorvorrichtung (30) dazu konfiguriert ist, mindestens einen Signalvorhang (34), vorzugsweise Licht- oder Laservorhang; und/oder mindestens eine Signalschranke, vorzugsweise Licht- oder Laserschranke; und/oder mindestens ein Signalgitter, vorzugsweise Lichtgitter oder Lasergitter, zum Erfassen des Eindringens zu erzeugen.

10. Behälterbehandlungsanlage (10) nach Anspruch 9, wobei:
die Sensorvorrichtung (30) dazu konfiguriert ist, mehrere Signalvorhänge (38), mehrere Signalschranken oder mehrere Signalgitter derart zu erzeugen, dass die mehreren Signalvorhänge, die mehreren Signalschranken oder die mehreren Signalgitter winkelig zueinander angeordnet sind und aneinander angrenzen oder sich schneiden.

11. Behälterbehandlungsanlage (10) nach einem der vorherigen Ansprüche, wobei die Sensorvorrichtung (30) aufweist:
einen Emitter, vorzugsweise Laseremitter, wobei der Emitter dazu konfiguriert ist, einen, vorzugsweise kreisförmigen oder fächerförmigen, Signalvorhang (38), vorzugsweise Laservorhang, auszugeben; und
einen Empfänger, vorzugsweise Laserempfänger, wobei der Empfänger dazu konfiguriert ist, zu erfassen, wenn ein Signalstrahl, vorzugsweise Laserstrahl, des Signalvorhangs (38) an einem Objekt rückreflektiert wird.

12. Behälterbehandlungsanlage (10) nach einem der vorherigen Ansprüche, wobei:
die Verarbeitungsvorrichtung (38) dazu konfiguriert ist, den Betrieb abhängig von der Signalausgabe derart anzupassen, dass ein automatischer Umrüst-, Teilewechsel- oder Wartungsbetrieb der Behälterbehandlungsvorrichtung (12) gestoppt wird, wenn die Signalausgabe der Sensorvorrichtung (30) das Eindringen in den vorgegebenen Schutzbereich (32) angibt,
wobei vorzugsweise in dem automatischen Umrüst-, Teilewechsel- oder Wartungsbetrieb ein öffenbarer Abschnitt (18) der Schutzeinhausung (16) geöffnet ist.

13. Behälterbehandlungsanlage (10) nach einem der vorherigen Ansprüche, wobei:
die Verarbeitungsvorrichtung (38) dazu konfiguriert ist, dass die Sensorvorrichtung (30), der Schutzbereich (32) und/oder eine Verarbeitung der Signalausgabe der Sensorvorrichtung (30) deaktiviert ist, wenn die Behälterbehandlungsvorrichtung (12) in einem Betriebsmodus betrieben wird, in dem die Schutzeinhausung (16) und/oder ein öffenbarer Abschnitt (18) der Schutzeinhausung (16) geschlossen ist.

14. Behälterbehandlungsanlage (10) nach einem der vorherigen Ansprüche, wobei:
die Sensorvorrichtung (30) dazu konfiguriert ist, ein Eindringen und/oder Aufenthalt in einem vorgegebenen sicheren Zugangskorridor (36) in dem vorgegebenen Schutzbereich (32) zu erfassen; oder
die Sensorvorrichtung (30) dazu konfiguriert ist, ein Eindringen und/oder Aufenthalt in einem vorgegebenen sicheren Zugangskorridor (36) in dem vorgegebenen Schutzbereich (32) nicht als ein Eindringen in den vorgegebenen Schutzbereich (32) zu erfassen.

15. Behälterbehandlungsanlage (10) nach einem der vorherigen Ansprüche, ferner aufweisend:
mindestens eine weitere Behälterbehandlungsvorrichtung (22, 24, 26, 28), die neben der Behälterbehandlungsvorrichtung (12) angeordnet ist,
wobei die Sensorvorrichtung (30) dazu konfiguriert ist, dass der Schutzbereich (32) derart vorgegeben ist, dass die mindestens eine weitere Behälterbehandlungsvorrichtung (22, 24, 26, 28) den Schutzbereich (32) begrenzt.
